# EUROPÄISCHE PATENTSCHRIFT

(11) **EP 0 334 289 B1**
(45) Veröffentlichungstag und Bekanntmachung des Hinweises auf die Patenterteilung: **16.06.1993**
(21) Anmeldenummer: 89105039.5
(22) Anmeldetag: 21.03.1989
(51) Int. Cl.: C07D 498/04, C07D 498/10, A61K 31/42

(54) **Heterocyclisch substituierte 5,7-Dihydro-pyrrolo[3,2-f]benzoxazol-6-one, Verfahren zu ihrer Herstellung sowie diese Verbindungen enthaltende Arzneimittel**
Heterocyclic substituted 5,7-dihydropyrrolo (3,2-f) benzoxazol-6-ones, process for their preparation and drugs containing same
5,7-Dihydropyrrolo (3,2-f) benzoxazol-6-ones substituées par hétérocycle, procédé pour leur préparation et médicaments les contenant

(30) Priorität: 24.03.1988 DE 3809868
(43) Veröffentlichungstag der Anmeldung: 27.09.1989
(73) Patentinhaber: BOEHRINGER MANNHEIM GMBH, 68298 Mannheim (DE)
(72) Erfinder: von der Saal, Wolfgang, Dr.rer.nat., D-6940 Weinheim (DE); Haag, Rainer, Dr.rer.nat., D-6800 Mannheim 25 (DE)

(56) Entgegenhaltungen:
- EP-A- 0 189 103
- EP-A- 0 214 592

## Beschreibung

Die vorliegende Erfindung betrifft neue Verbindungen der allgemeinen Formel I, 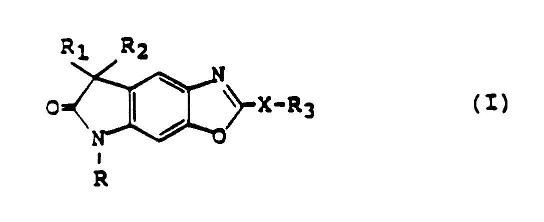
in der
- R: ein Wasserstoffatom oder eine Alkylgruppe darstellt,
- R₁: ein Wasserstoffatom, eine Alkyl-, Alkenyl- oder eine Cycloalkylgruppe bedeutet,
- R₂: ein Wasserstoffatom, eine Alkyl-, Alkenyl-, Alkylcarbonyl-, Alkoxycarbonyl-, Aminocarbonyl- oder Hydrazinocarbonylgruppe bedeutet, oder R₁ und R₂ zusammen mit dem C-Atom, an das sie gebunden sind, einen Cycloalkylring bilden,
- X: eine Bindung, eine Alkylen- oder die Vinylengruppe bedeutet,
- R₃: einen aromatischen heterocyclischen Fünfring mit 1-4 Heteroatomen oder einen aromatischen heterocyclischen Sechsring mit 1-5 Heteroatomen darstellt, wobei die Heteroatome gleich oder verschieden sein können und Sauerstoff, Schwefel oder Stickstoff bedeuten und gewünschtenfalls an einem oder mehreren Stickstoffatomen ein Sauerstoffatom tragen können, und die Fünf- oder Sechringe gegebenenfalls durch eine oder mehrere Alkyl-, Alkoxy-, Alkoxycarbonyl-, Carboxyl-, Alkylmercapto-, Hydroxy-, Nitro-, Amino-, Halogen- oder Cyangruppen substituiert und gewünschtenfalls mit einem Phenylring oder einem aromatischen Fünf- oder Sechsring mit 1-4 Heteroatomen zu einem Bicyclus kondensiert sein können,
deren Tautomere, und optisch aktiven Formen und deren physiologisch verträgliche Salze anorganischer oder organischer Säuren und Verfahren zu ihrer Herstellung, sowie diese Verbindungen enthaltende Arzneimittel.

Für den Fall, daß die Verbindungen der allgemeinen Formel I ein asymmetrisches Atom enthalten, sind auch die optisch aktiven Verbindungen und racemischen Gemische Gegenstand der Erfindung. Die optisch aktiven Verbindungen können aus den racemischen Gemischen nach an sich bekannten Methoden über diastereomere Salze hergestellt werden. Zur Racematspaltung können optisch aktive Salze oder Basen, wie z.B. Weinsäure, Äpfelsäure oder Camphersulfonsäure, verwendet werden.

Substituierte 5,7-Dihydro-pyrrolo[3,2-f]benzoxazol-6-on-Derivate mit blutdrucksenkender Wirkung sind aus EP-A-214 592 bekannt.

Die neuen Verbindungen der allgemeinen Formel I weisen wertvolle pharmakologische Eigenschaften auf. Insbesondere können sie als Immunsuppressiva bei Immunerkrankungen, z.B. Rheumatoide Arthritis, Diabetes Mellitus Typ I, Psoriasis, Lupus Systemicus Erythematosus etc., sowie zur Therapie von Abstoßungsreaktionen nach Organ/Gewebetransplantationen (z.B. von Haut, Knochenmark, Nieren etc.) eingesetzt werden. Ferner können sie bei al jenen Erkrankungen therapeutisch Verwendung finden, bei denen eine polyklonale B-Zell-Aktivierung/Proliferation von pathophysiologischer, symptomatischer und/oder klinischer Relevanz sein könnte, d.h. neben Autoimmunerkrankungen auch bei ARC/AIDS sowie bei viralen Infektionen jeglicher Genese.

Die oben genannten Verbindungen wirken ferner zytostatisch/zytotoxisch und eignen sich daher insbesondere für die Behandlung von B-Zell/T-Zell/Plasma-Zell-Leukämien bzw. Neoplasien, wie z.B. chronisch lymphatische Leukämie, lymphoblastische Lymphome, Multiple Myelome etc..

In der allgemeinen Formel I können die Substituenten R₁ und R₂ gleich oder verschieden sein und Wasserstoff, eine geradkettige oder verzweigte, gesättigte oder ungesättigte Alkylgruppe mit 1-6 bzw. 2-6 Kohlenstoffatomen, eine durch Alkyl-, Alkoxy-, Amino- oder Hydrazinogruppe substituierte Carbonylgruppe darstellen, wobei jeder Alkylrest geradkettig oder verzweigt aus 1-6 bzw. 2-6 Kohlenstoffatomen zusammengesetzt sein kann.

Insbesondere kommen jedoch Wasserstoff, die Methyl-, Ethyl-, Allyl-, Acetyl-, Propionyl-, Methoxycarbonyl-, Ethoxycarbonyl-, und Hydrazinocarbonylgruppe für R₁ und R₂ in Frage.

Stellt nur R₂ ein Wasserstoffatom dar, so ist R₁ insbesondere eine geradkettige Alkylgruppe mit 1-6 Kohlenstoffatomen oder eine verzweigte Alkylgruppe bzw. eine Cycloalkylgruppe mit 3-7 Kohlenstoffatomen, eine durch eine Alkyl-, Alkoxy-, Amino- oder Hydrazinogruppe substituierte Carbonylgruppe. Bevorzugt in diesem Sinne sind die Methyl-, Ethyl-, Isopropyl-, Isobutyl-, Pentyl-, Cyclopentyl-, Cyclohexyl-, Allyl-, Acetyl-, Propionyl-, Methoxycarbonyl-, Ethoxycarbonyl-, und Hydrazinocarbonylgruppe.

R₁ und R₂ können zusammen mit dem C-Atom, an das sie gebunden sind, auch einen Cycloalkylring mit 3-8 Kohlenstoffatomen bilden, vorzugsweise handelt es sich dabei um die Spirocyclopropyl-, Spirocyclobutyl-, Spirocyclopentyl, und Spirocyclohexylgruppe.

R bedeutet bevorzugt ein Wasserstoffatom, kann aber auch eine C₁-C₆-Alkylgruppe, wie z.B. eine Methyl-, Ethyl-, oder Isopropylgruppe darstellen.

Bedeutet in der allgemeinen Formel I X eine Alkylengruppe, so sind darunter Ketten mit 1-6 C-Atomen zu verstehen, die auch verzweigt sein können.

Besonders bevorzugt sind geradkettige Gruppen, wie z.B. die Methylen-, Ethylen-, Propylen- und Butylengruppe.

Die bei R₃ angegebenen heterocyclischen Fünfringe mit 1-4 Heteroatomen oder heterocyclischen Sechsringe mit 1-5 Heteroatomen, wobei die Heteroatome der vorgenannten Fünf- oder Sechsringe gleich oder verschieden sein können und Stickstoff, Sauerstoff oder Schwefel bedeuten und gegebenenfalls an einem oder mehreren Stickstoffatomen ein Sauerstoff tragen können, bedeuten bevorzugt den Pyrrol-, Furan-, Thiophen-, Pyrazol-, Imidazol-, Thiazol-, Isothiazol-, Oxazol-, Isoxazol-, Triazol-, Thiadiazol-, Oxadiazol-, Pyrazin-, N,N′-Dioxy-pyrazin-, Pyrimidin-, N,N′-Dioxy-pyrimidin-, Pyridazin-, Oxazin-, Thiazin-, Triazin-, Tetrazin-, Pyridin- und den N-Oxy-pyridinrest.

Alkyl-, Alkoxy- und Alkylmercapto-Substituenten in den heterocyclischen Fünf- und Sechsring können 1-6, vorzugsweise 1-4 Kohlenstoffatome enthalten. Bevorzugt ist der Methyl-, Ethyl-, Methoxy-, Ethoxy-, Methylmercapto- und Ethylmercaptorest. Unter Halogen ist Fluor, Chlor und Brom, vorzugsweise Chlor zu verstehen.

Sind die aromatischen heterocyclischen Fünf- und Sechsringe mit einem Phenylring kondensiert so sind der Indol-, Indazol-, Benzimidazol-, Chinolin-, Isochinolin-, Cinnolin-, Phthalazin-, Chinazolin-, Chinoxalin-, Benzofuran-, Benzothiophen-, Benzoxazol-, Benzoisoxazol-, Benzothiazol-, Benzoisothiazol, Benzotriazol- und der Benzothiadiazolrest bevorzugt.

Sind die aromatischen heterocyclischen Fünf- und Sechsringe mit einem weiteren aromatischen heterocyclischen Fünf- oder Sechsring zu einem Bicyclus kondensiert, so sind darunter bevorzugt der Naphthyridin-, Pteridin-, Purin-, Indolizon-, Thiopheno[2,3-b]pyrazin- und der Imidazol[1,2-a]pyridinrest.

Besonders bevorzugte Verbindungen der allgemeinen Formel I sind Verbindungen, in denen
R ein Wasserstoffatom darstellt,
R₁ und R₂ gleich sind und C₁-C₄-Alkylgruppen, insbesondere Methylgruppen bedeuten oder R₁ und R₂ zusammen mit dem Kohlenstoffatom, an das sie gebunden sind, einen Spirocyclopentylring bilden,
- X: eine Bindung, eine C₁-C₄-Alkylen- oder Vinylengruppe darstellt und
- R₃: den Pyrrolyl-, Furanyl-, Thienyl-, Pyrazolyl-, Imidazolyl-, Isothiazolyl-, Thiazolyl-, Oxazolyl-, Triazolyl-, Tetrazolyl-, Thiadiazolyl-, Isoxazolyl-, Oxadiazolyl-, Pyridinyl-, N-Oxy-pyridinyl-, Pyrazinyl-, N,N′-Dioxy-pyrazinyl-, Pyrimidinyl-, N,N′-Dioxypyrimidinyl-, Pyridazinyl-, Oxazinyl-, Thiazinyl-, Triazinyl- oder Tetrazinylrest darstellt, sowie deren C₁-C₄-Alkyl-, wie z.B. Methyl-, Ethyl-; C₁-C₄-Alkoxy-, wie z.B. Methoxy-, Ethoxy-; C₁-C₄-Alkylmercapto-, wie z.B. Methylmercapto-, Ethylmercapto-; und Halogen-, wie z.B. Chlor substituierten Derivate, oder R₃ den Indolyl-, Indazolyl-, Chinolinyl-, Isochinolinyl- oder Imidazo[1,2-a]pyridinylrest bedeutet.

Die Verbindungen der allgemeinen Formel I und deren Tautomere können nach an sich bekannten Verfahren hergestellt werden. Besonders vorteilhaft sind die in den Schemata 1-3 gezeigten Verfahren.

Das in Schema 1 dargestellte Verfahren geht aus von 1,3-Dihydro-6-hydroxy-2H-indol-2-onen der allgemeinen Formel II, in der R₁ und R₂ die oben angegebenen Bedeutungen haben. Diese Verbindungen sind bekannt oder können in Analogie zu den Vorschriften in der Internationalen Patentanmeldung WO 83/02610 und in Th. Wieland, O, Unger, Chem. Ber. 96 (1963) 253, hergestellt werden.

Die weiteren Stufen 1-4 des Schemas 1 folgen bekannten Methoden der Benzoxazolsynthese, wie sie in J.W. Cornforth in Heterocyclic Compounds (R.C. Elderfield, Editor) Vol. 5, J. Wiley + Sons, New York 1957, S. 418 ff beschrieben sind. Es handelt sich um folgende Basisverfahren: (1 a) und (2 c) Nitrierung; (1 b) und (2 b) Veresterung; (2 a) und (3 c) Reduktion; (3 a) Bildung einer Schiffschen Base durch Umsetzung mit einem Aldehyd; (3 b) Ringschluß durch Erhitzen mit einem Carbonsäurederivat; (4) Ringschluß durch Oxidation.

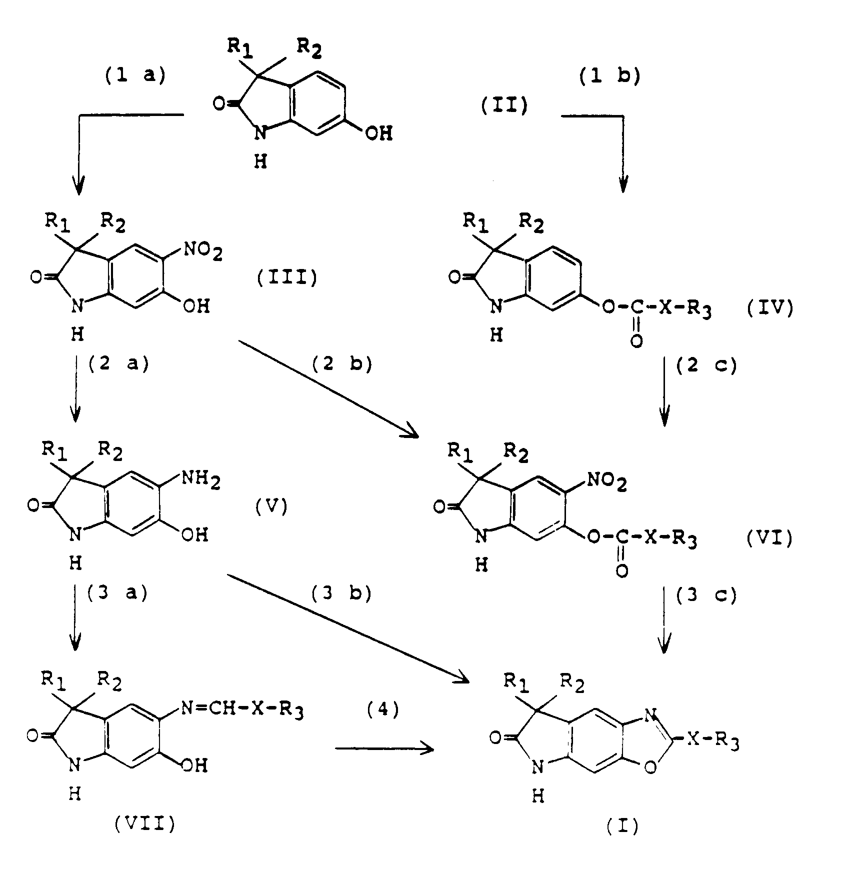

### Stufe 1 a

Durch Nitrierung von Verbindungen der allgemeinen Formel II erhält man Verbindungen der allgemeinen Formel III, in der R₁ und R₂ die oben angegebenen Bedeutungen haben. Die Nitrierung führt man bevorzugt mit Salpetersäure in Schwefelsäure bei Temperaturen zwischen -20°C und +50°C durch. Sie kann jedoch auch ohne Schwefelsäure, oder an deren Stelle in Wasser, Eisessig oder Acetanhydrid durchgeführt werden, oder mit N₂O₅ in CCl₄ in Gegenwart von P₂O₅. Als Nitrierungsreagenzien können auch Anhydride wie Acetylnitrat, oder Nitrylhalogenide mit FeCl₃, Methylnitrat und BF₃, oder Nitroniumsalze wie NO₂BF₄, NO₂PF₆ oder NO₂CF₃SO₃ dienen. Zur Nitrierung kann auch eine Mischung aus Salpetersäure und salpetriger Säure benutzt werden, welche N₂O₄ als eigentliche nitrierende Spezies liefert.

### Stufe 1 b

Verbindungen der allgemeinen Formel IV, in der R₁, R₂, R₃ und X die oben genannte Bedeutung haben, stellt man aus Verbindungen der allgemeinen Formel II her durch Umsetzung mit den Carbonsäuren oder davon abgeleiteten Derivaten der allgemeinen Formel VIII,

Y-X-R₃ (VIII)

in der R₃ und X die oben angegebenen Bedeutungen haben und Y die Carboxylgruppe, die Alkoxycarbonylgruppe, die Alkoxycarbonyloxycarbonylgruppe oder die Halogencarbonylgruppe bedeutet. Bedeutet Y die Carboxylgruppe, dann findet die Umsetzung zwischen dem Phenol der allgemeinen Formel II und der Carbonsäure der allgemeinen Formel VIII in einem inerten Lösungsmittel wie Dichlormethan, Toluol, Xylol, DMF, bei Temperaturen zwischen 50°C und 150°C, vorzugsweise beim Siedepunkt des Lösungsmittels statt. Das entstehende Wasser entfernt man entweder durch azeotrope Destillation, oder durch ein Kondensationsmittel wie Phosphoroxychlorid, Thionylchlorid, Schwefelsäure, Phosphorsäure oder Molekularsieb. Andere Reagenzien, die die Reaktion beschleunigen, sind Dicyclohexylcarbodiimid, N,N′-Carbonyldiimidazol, BF₃, Trifluoressigsäureanhydrid, H₃BO₃/H₂SO₄ und polymergebundenes Aluminiumchlorid.

### Stufe 2 a:

Verbindungen der allgemeinen Formel III werden in Verbindungen der allgemeinen Formel V überführt durch Reduktion der Nitrogruppe.

Die Reduktion wird vorzugsweise in einem Lösungsmittel oder Lösungsmittelgemisch wie Wasser, Methanol, Ethanol, Eisessig, Essigsäureethylester oder Dimethylformamid mit Wasserstoff in Gegenwart eines Katalysators wie Raney-Nickel, Platin oder Palladium/Kohle, mit Metallen wie Eisen, Zinn oder Zink in Gegenwart einer Säure, mit Salzen wie Eisen(II)sulfat, Zinn(II)chlorid, Natriumsulfid, Natriumhydrogensulfit oder Natriumdithionit mit Hydrazin in Gegenwart von Raney-Nickel bei Temperaturen zwischen 0 und 100°C, vorzugsweise jedoch bei Raumtemperatur, durchgeführt. Eine Isolierung von Verbindungen der allgemeinen Formel V kann jedoch auch unterbleiben. Die Lösungen werden dann wie in Stufe 3 a beschrieben weiter umgesetzt.

### Stufe 2 b:

Die Verbindungen der allgemeinen Formel III können durch Veresterung in Verbindungen der allgemeinen Formel VI überführt werden. Die Veresterungen werden wie in Stufe 1 b beschrieben, durchgeführt.

### Stufe 2 c:

Die Verbindungen der allgemeinen Formel IV können durch Nitrierung in Verbindungen der allgemeinen Formel VI überführt werden. Die Nitrierung wird wie in Stufe 1 a beschrieben durchgeführt.

### Stufe 3 a:

Die Verbindungen der allgemeinen Formel V werden in Verbindungen der allgemeinen Formel VII überführt, indem man sie mit Aldehyden der allgemeinen Formel IX umsetzt,

OHC-X-R₃ (IX)

in der R₃ und X die oben genannte Bedeutung haben. Dazu werden die Verbindungen in einem inerten Lösungsmittel wie Dichlormethan, Toluol, Xylol, Chlorbenzol oder Ether zusammengegeben und durch Abziehen des Lösungsmittels isoliert. Eine bervorzugte Methode ist jedoch, Verbindungen der allgemeinen Formel VII nicht zu isolieren, sondern gleich zu Verbindungen der allgemeinen Formel I weiter umzusetzen (siehe Stufe 4).

### Stufe 3 b

Die Verbindungen der allgemeinen Formel V können zu Verbindungen der allgemeinen Formel I cyclisiert werden, indem man sie mit Carbonsäuren oder deren Derivaten der allgemeinen Formel X

Z-X-R₃ (X)

umsetzt, wobei X und R₃ die oben genannten Bedeutungen besitzen, und Z die Carboxylgruppe, die Alkoxycarbonylgruppe, die Alkoxycarbonyloxycarbonylgruppe, die Chlorcarbonylgruppe, die Aminocarbonylgruppe und die Nitrilgruppe bedeuten kann. Die Umsetzungen können ohne Lösungsmittel, d.h. in der Schmelze bei Temperaturen zwischen 150°C und 250°C durchgeführt werden.

Ist die Verbindung der allgemeinen Formel X eine Carbonsäure, so findet die Umsetzung mit Verbindungen der allgemeinen Formel V in Gegenwart eines wasserentziehenden Mittels, vorzugsweise in Polyphosphorsäure bei Temperaturen zwischen 50 und 250°C, vorzugsweise zwischen 100 und 200°C statt.

Ist die Verbindung der allgemeinen Formel X ein Carbonsäurederivat, so findet die Umsetzung mit Verbindungen der allgemeinen Formel V in einem inerten Lösungsmittel, vorzugsweise in Methylenchlorid oder Pyridin statt. Zur Vervollständigung der Cyclisierung erhitzt man anschließend in einem Lösungsmittel oder Lösungsmittelgemisch wie Ethanol, Isopropanol, Eisessig, Benzol, Chlorbenzol, Glycol, Diethylenglycoldimethylether, Sulfolan oder Dimethylformamid auf Temperaturen zwischen 50 und 250°C, vorzugsweise jedoch auf die Siedetemperatur des Lösungsmittels oder Lösungsmittelgemisches, gegebenenfalls in Gegenwart einen Kondensationsmittels wie Phosphoroxychlorid, Thionylchlorid, p-Toluolsulfonsäure, Salzsäure, Schwefelsäure, Phosphorsäure, Polyphosphorsäure oder gegebenenfalls auch in Gegenwart einer Base wie Natriumhydroxid, Kaliummethylat oder Kalium-tert.butylat.

### Stufe 3 c:

Die Reduktion von Verbindungen der allgemeinen Formel VI führt direkt zum Ringschluß, und man isoliert die Verbindungen der allgemeinen Formel I. Die Reduktion führt man durch wie bei Stufe 2 a beschrieben.

### Stufe 4:

Aus den Schiffschen Basen der allgemeinen Formel VII erhält man durch Oxidation die Verbindungen der allgemeinen Formel I. Die Reaktion führt man durch vorzugsweise in alkoholischem Medium unter Erwärmen zum Rückfluß in Gegenwart von Luftsauerstoff und katalytischen Mengen Säure, wie Toluolsulfonsäure, oder in Gegenwart von Luftsauerstoff und eines Katalysators wie Braunstein in saurem Milieu wie z.B. in Eisessig bei Raumtemperatur, oder mit Bleitetraacetat, Chloranil, N-Bromsuccinimid, N-Jodsuccinimid, Sulfurylchlorid, Wasserstoffperoxid, oder Eisenhexacyanoferrat.

Das besonders bevorzugte Verfahren zur Herstellung von Verbindungen der allgemeinen Formel I aus Verbindungen der allgemeinen Formel II besteht in der Nitrierung (Stufe 1 a) und anschließenden Reduktion (Stufe 2 a). Die Verbindungen der allgemeinen Formel V werden nun nicht isoliert, sondern unmittelbar mit den Aldehyden der allgemeinen Formel IX (Stufe 3 a) in Gegenwart eines Oxidationsmittels (Stufe 4), oder mit den Carbonsäurederivaten der allgemeinen Formel X (Stufe 3 b) umgesetzt.

Ein weiteres bevorzugtes Verfahren, ausgehend von den 5-Methyl-6-nitrobenzoxazolen der allgemeinen Formel XI, in der R₃ und X die oben genannten Bedeutungen haben, ist in Schema 2 darstellt.

Verbindungen der allgemeinen Formel XI sind bekannt: R.D. Haugwitz et al., J.Med.Chem. 25 (1982) 969-974, oder können nach den in dieser Literaturstelle genannten Vorschriften hergestellt werden. Der im Schema 2 dargestellten Syntheseweg folgt einem an sich bekannten Verfahren (Reissert-Synthese) zur Herstellung von 1,3-Dihydro-2H-indol-2-onen, wie es beispielsweise in der Internationalen Patentanmeldung WO 83/02610 (R.Anchi, Sandoz AG, Basel, 19.1.83) beschrieben ist. Es umfaßt folgende Basisverfahren: (5) Umsetzung mit Oxalsäurediethylester in Gegenwart einer Base; (6 a) Alkylierung oder Acylierung; (6 b) und (7 a) alkalische Hydrolyse; (8 a) und (7 b) Reaktion mit H₂O₂; (9 a) und (8 b) katalytische Hydrierung und Erhitzen; (9 b) Alkylierung oder Acylierung.

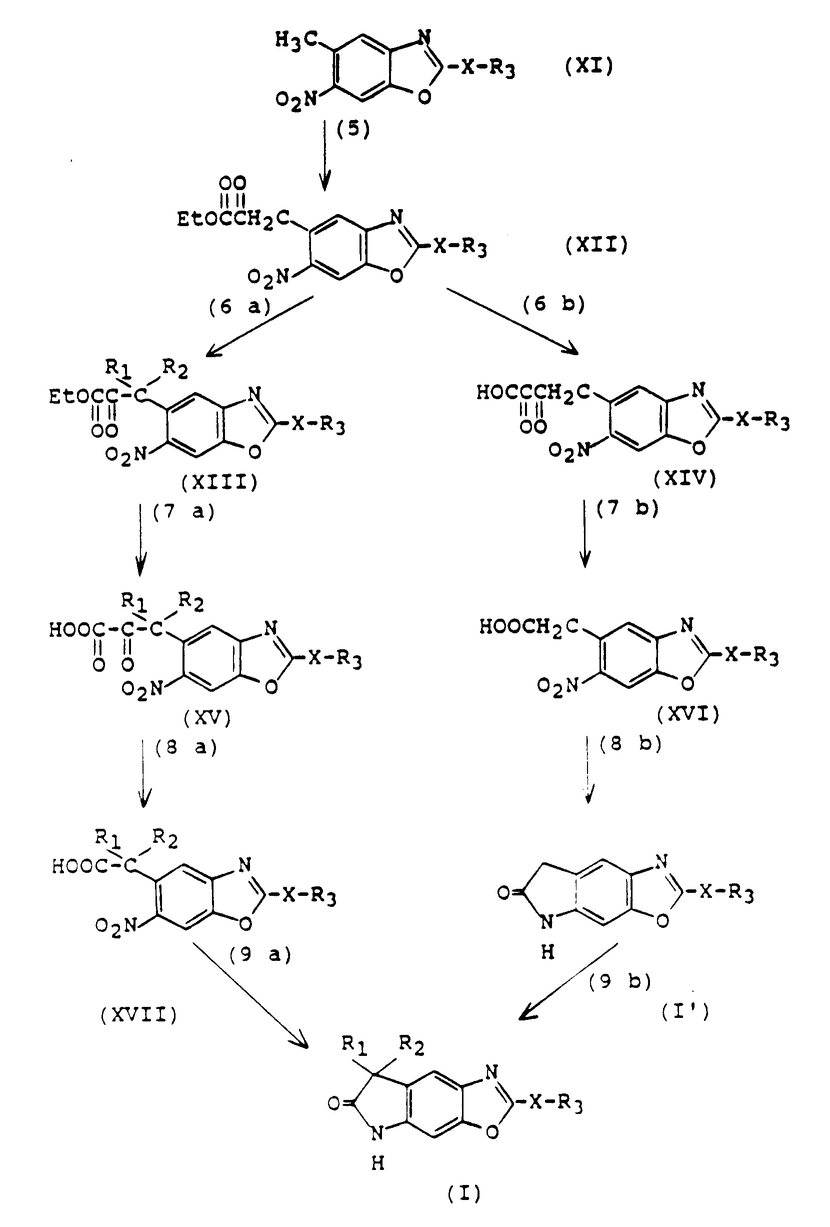

### Stufe 5:

Durch Umsetzung von Verbindungen der allgemeinen Formel XI mit Oxalsäurediethylester erhält man Verbindungen der allgemeinen Formel XII, in der R₃ und X die oben angegebene Bedeutung aufweisen. Diese Umsetzung führt man in einem inerten Lösungsmittel wie Dichlormethan, Ether, Toluol oder Xylol durch in Gegenwart einer Base wie Kalium- oder Natriummethylat oder -ethylat oder Kalium-tert.butylat, bei Temperaturen zwischen -20°C und 50°C, vorzugsweise bei Raumtemperatur durch. Die Verbindungen der allgemeinen Formel XII fallen nach einiger Zeit in Form der Natrium- oder Kaliumsalze aus, oder werden durch Eindampfen des Lösungsmittels gewonnen.

### Stufe 6 a:

Durch Alkylierung erhält man aus den Verbindungen der allgemeinen Formel XII die Verbindungen der allgemeinen Formel XIII. Diese Alkylierungen führt man in einem inerten Lösungsmittel wie Dichloromethan, Ether, Toluol oder Xylol in Gegenwart einer Base wie Natronlauge, Kalilauge, gewünschtenfalls in Gegenwart eines Phasentransferkatalysators, mit einem Alkylierungsmittel durch, das die Reste R₁ und R₂ überträgt, durch. Als Alkylierungsmittel kommen Alkylhalogenide oder Alkylsulfate in Betracht.

### Stufe 6 b:

Die Verbindungen der allgemeinen Formel XII werden alkalisch verseift zur Verbindungen der allgemeinen Formel XIV, in der X und R₃ die oben angegebenen Bedeutungen besitzen. Diese Verseifung führt man am besten in Wasser oder einem Alkohol oder einer Mischung aus beiden in Gegenwart einer Base wie Natronlauge oder Kalilauge bei Temperaturen zwischen 0°C und 100°C, vorzugsweise bei Raumtemperatur durch.

### Stufe 7 a:

Durch alkalische Verseifung, wie in Stufe 6 b beschrieben, erhält man die Verbindungen der allgemeinen Formel XV, in der X und R₃ die oben angegebene Bedeutung besitzen. Diese Stufe kann auch übergangen werden, wenn man Verbindungen der allgemeinen Formel XIII unmittelbar, wie in Stufe 8 a beschrieben, oxidiert.

### Stufe 7 b:

Verbindungen der allgemeinen Formel XII oder XIV oxidiert man zu Verbindungen der allgemeinen Formel XVI, in der X und R₃ die oben angegebenen Bedeutungen besitzen.

Bevorzugt werden die Verbindungen der allgemeinen Formel XII oder XIV in Wasser oder einem Alkohol gelöst und in Gegenwart von Base, wie Natronlauge oder Kalilauge mit Wasserstoffperoxid bei Temperaturen zwischen -20°C und +50°C, vorzugsweise bei Raumtemperatur oxidiert. Nach Ansäuern fallen im allgemeinen die Verbindungen der allgemeinen Formel XVI aus der Lösung aus, oder sie werden durch Eindampfen der Lösung gewonnen.

### Stufe 8 a:

Verbindungen der allgemeinen Formel XVII erhält man durch Oxidation von Verbindungen der allgemeinen Formel XIII oder XV. Das Verfahren wird wie für Stufe 7 b beschrieben durchgeführt.

### Stufe 8 b:

Verbindungen der allgemeinen Formel I′, in der X und R₃ die oben angegebene Bedeutung haben, erhält man aus Verbindungen der allgemeinen Formel XVI durch Reduktion der Nitrogruppe und anschließendem Erhitzen. Die Verbindungen der allgemeinen Formel I′ unterscheiden sich von denen der allgemeinen Formel I dadurch, daß R₁ und R₃ jeweils Wasserstoffatome bedeuten. Die Reduktion der Nitrogruppe führt man durch wie in Stufe 2 a beschrieben. Dabei tritt teilweise unmittelbar die Cyclisierung zu Verbindungen der allgemeinen Formel I′ ein.

Die Cyclisierung kann gewünschtenfalls vervollständigt werden, indem man nach der Reduktion vorzugsweise in einem Lösungsmittel wie Ethanol, Isopropanol, Eisessig, Benzol, Toluol, Chlorbenzol, Glycol, Ethylenglycoldimethylether, Sulfolan, Dimethylformamid oder Gemischen dieser Lösungsmittel auf Temperaturen zwischen 50 und 220°C, vorzugsweise jedoch auf die Siedetemperatur des Reaktionsgemisches, gegebenenfalls in Gegenwart eines Kondensationsmittels wie Phosphoroxychlorid, Thionylchlorid, p-Toluolsulfonsäure, Salzsäure, Schwefelsäure, Phosphorsäure, Polyphosphorsäure oder gegebenenfalls auch in Gegenwart einer Base wie Natriumhydroxid, Natriumethylat oder Kalium-tert.butylat erhitzt. Die Cyclisierung kann jedoch auch ohne Lösungsmittel und/oder Kondensationsmittel durchgeführt werden.

### Stufe 9 a:

Die Verbindungen der allgemeinen Formel XVII werden reduziert und nach dem Verfahren, wie es für Stufe 8 b beschrieben ist, zu Verbindungen der allgemeinen Formel I cyclisiert.

### Stufe 9 b:

Verbindungen der allgemeinen Formel I, in der R₃, und X die oben genannte Bedeutung haben, und in der R₁ und R₂ Wasserstoffatome bedeuten (= allgemeine Formel I′), können in Verbindungen der allgemeinen Formel I überführt werden, in denen R₁, R₂, R₃ und X die oben genannten Bedeutungen besitzen. Dazu ist es notwendig, das freie NH-Proton durch eine Schutzgruppe, bevorzugt durch die Acetylgruppe, zu ersetzen. Anschließend führt man die Alkylierung oder Acylierung wie bei Stufe 6 a beschrieben durch, und spaltet die Acetyl-Schutzgruppe ab.

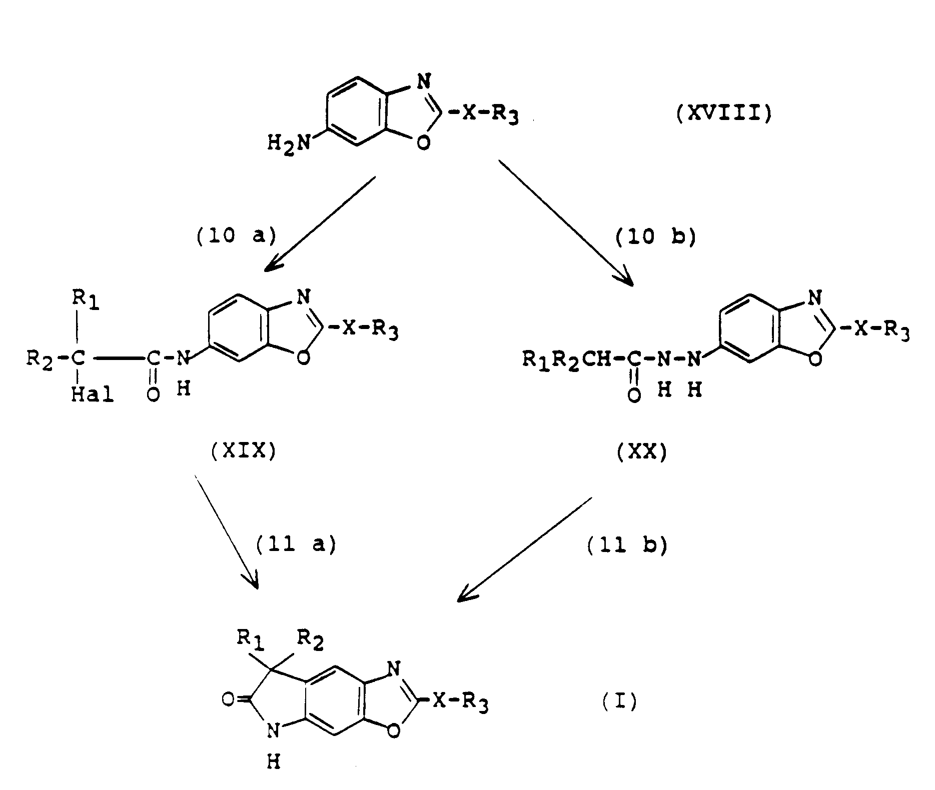

Ein drittes bevorzugtes Verfahren geht, wie Schema 3 zeigt, aus von den 6-Amino-benzoxazolen der allgemeinen Formel XVIII, in der X und R₃ die oben genannten Bedeutungen besitzen. Die Verbindungen der allgemeinen Formel XVIII sind literaturbekannt (R. Haugwitz et al., J. Med. Chem. 25 (1982) 969 ff) oder können nach den darin beschriebenen Verfahren hergestellt werden.

Bei den Verfahren in Schema 3 handelt es sich um folgende Basisverfahren: (10 a) und (11 a) folgen der Oxindolsynthese nach Stolle, wie sie in P.L. Julian, E. W. Meyer, H.C. Printy, (R.C. Elderfield, Editor), Heterocyclic Compounds, Vol. 3, Wiley and Sons, New York 1952, S. 142 ff, beschrieben ist. (10 b) und (11 b) folgen der Oxindolsynthese nach Brunner, wie sie beschrieben ist in ibid, S. 141 ff.

### Stufe 10 a:

Verbindungen der allgemeinen Formel XVIII werden acyliert durch alpha-Halogencarbonsäurederivaten, vorzugsweise durch alpha-Halogensäurechloride oder -bromide der allgemeinen Formel XXI 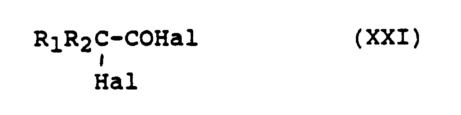
in der R₁ und R₂ die oben angegebene Bedeutung besitzen und Hal ein Halogenatom bedeutet. Man arbeitet im allgemeinen bei Temperaturen zwischen -10°C und Raumtemperatur. Bevorzugt geht man dabei so vor, daß nach Schotten-Baumann zur wässrigen Lösung des Amins, welche noch eine Base wie Alkalihydroxid, Natriumcarbonat oder Pyridin enthält, das Säurechlorid unter Eiskühlung langsam zutropft und anschließend noch einige Zeit bei Raumtemperatur stehen läßt. Diese Reaktion ist nicht nur in Wasser möglich, sondern auch in organischen Lösungsmitteln wie Methylenchlorid, Ether, Benzol oder Toluol. Auch ohne säurebindende Mittel lassen sich die Amine durch Carbonsäurechloride nahezu quantitativ acylieren, indem man das Amin und das Carbonsäurechlorid in einem inerten Lösungsmittel wie Methylenchlorid, Benzol oder Toluol bis zur Beendigung der Gasentwicklung kocht, was 1-24 Stunden dauert. Gibt man jedoch ein säurebindendes Mittel wie Triethylamin oder Pyridin in geringem Überschuß zu, so läuft die Reaktion schon bei Temperaturen zwischen -10°C und Raumtemperatur ab.

### Stufe 10 b:

Aus den Verbindungen der allgemeinen Formel XVIII erhält man die Verbindungen der allgemeinen Formel XX, in der R₁, R₂, R₃ und X die oben angegebenen Bedeutungen haben, in einem dreistufigen Eintopfverfahren, indem man die Verbindungen der allgemeinen Formel XVIII diazotiert, die Diazoniumgruppe zum Hydrazin reduziert und dieses mit einem Säurechlorid der allgemeinen Formel XXII

R₁R₂CH-COHal (XXII),

in der R₁ und R₂ die oben angegebene Bedeutung haben und Hal ein Halogen bedeutet, reagieren läßt.

Die Diazotierung der Amine XVIII wird vorzugsweise unter neutralen oder sauren Bedingungen in einem polaren Lösungsmittel wie Wasser, Methanol, Ethanol, Eisessig, Salzsäure, Schwefelsäure, oder Phosphorsäure bei Temperaturen zwischen -70°C und 50°C vorzugsweise jedoch zwischen -5°C und 10°C durchgeführt.

Zur Diazotierung kommen vorwiegend anorganische Salze oder organische Ester der salpetrigen Säure in Frage wie z.B. Natrium- oder Kaliumnitrit oder Amylnitrit.

Die Reduktion der Diazoniumsalze wird überwiegend in den oben genannten Lösungsmitteln, in denen die Diazotierung ausgeführt wurde, bei Temperaturen zwischen -50°C und dem Siedepunkt des Lösungsmittels vorgenommen, vorzugsweise jedoch zwischen 0°C und 80°C, wobei als Reduktionsmittel Alkalisulfite, Schwefeldioxid, Dithionite, Zinn(II)chlorid, Zinkstaub, Eisen, Natriumamalgam, Triphenylphosphine, Endiole oder auch eine elektrochemische Reduktion in Frage kommen.

Die Hydrazine werden nun acyliert, nach dem in Stufe 10 a beschriebenen Verfahren.

### Stufe 11 a:

Zum Ringschluß werden Verbindungen der allgemeinen Formel XIX fein mit Aluminiumchlorid verrieben und auf Temperaturen zwischen 100°C und 270°C erhitzt, oder man führt die Reaktionen in einem Lösungsmittel wie CS₂ oder Nitrobenzol durch. An Stelle von Aluminiumchlorid kann man auch Zinkchlorid und andere starke Säuren verwenden. Die Cyclisierung ist auch photochemisch möglich, in Analogie zu Heterocycles 8, 2551 (1977).

### Stufe 11 b:

Der Ringschluß von Hydraziden der allgemeinen Formel XX wird in basischem Milieu bei erhöhter Temperatur durchgeführt. Dazu werden die Hydrazide XX innig mit Kalk, Calciumcarbonat oder Natriumhydrid vermengt und auf Temperaturen zwischen 150°C und 250°C erhitzt. Die Reaktion kann auch in einem hochsiedenden Lösungsmittel wie Diphenylether durchgefürht werden.

Die nach einem der Verfahren erhaltene Verbindung der allgemeinen Formel I oder deren Tautomer kann anschließend gewünschtenfalls in eine andere Verbindung der allgemeinen Formel I umgewandelt und/oder in ein physiologisch verträgliches Salz einer organischen oder anorganischen Säure überführt werden.

Die Umwandlung von Verbindungen der allgemeinen Formel I zu anderen Verbindungen der allgemeinen Formel I trifft zum Beispiel zu
a) für die Hydrierung einer Vinylenverbindung (X = -CH=CH-) in eine entsprechende Ethylenverbindung (X = -CH₂-CH₂-). Die Hydrierung wird vorzugsweise in einem Lösungsmittel wie Wasser, Wasser/Ethanol, Methanol, Eisessig, Essigsäureethylester oder Dimethylformamid mit Wasserstoff in Gegenwart eines Hydrierungskatalysators wie Raney-Nickel, Platin oder Palladium/Kohle durchgeführt.
b) für die Oxidation eines Fünf- oder Sechsringes mit einem oder mehreren Stickstoffatomen zu den entsprechenden N-Oxiden. Die Oxidation wird zweckmäßig mit einem oder mehreren Äquivalenten des verwendeten Oxidationsmittels durchgeführt, z.B. mit Wasserstoffperoxid in Eisessig, Trifluoressigsäure, oder Ameisensäure bei 20 -100°C oder in Aceton bei 0 -60°C, mit einer Persäure wie Perameisensäure oder m-Chlorperbenzoesäure in Eisessig, Trifluoressigsäure, Methylenchlorid oder Chloroform bei Temperaturen zwischen 0 und 60°C.
c) für die Umwandlung einer Verbindung der allgemeinen Formel I, in der R₂ eine Alkoxycarbonylgruppe bedeutet, in eine Verbindung der allgemeinen Formel I, in der R₂ die Hydrazinocarbonylgruppe bedeutet.

Ferner können die so erhaltenen Verbindungen der allgemeinen Formel I anschließend gewünschtenfalls in ihre physiologisch verträglichen Säureadditionssalze mit anorganischen oder organischen Säuren überführt werden. Als Säuren kommen hierfür beispielsweise Salzsäure, Bromwasserstoffsäure, Schwefelsäure, Phosphorsäure, Fumarsäure, Bernsteinsäure, Weinsäure, Zitronensäure, Milchsäure, Maleinsäure oder Methansulfonsäure in Betracht.

Zur Herstellung von Arzneimitteln werden die Substanzen der allgemeinen Formel I in an sich bekannter Weise mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder Öl, wie z.B. Olivenöl, suspendiert oder gelöst.

Die erfindungsgemäßen neuen Substanzen der allgemeinen Formel I und ihre Salze können in flüssiger oder fester Form enteral oder parenteral appliziert werden. Als Injektionsmedium kommt vorzugsweise Wasser zur Anwendung, welches die bei Injektionslösungen üblichen Zusätze wie Stabilisierungsmittel, Lösungsvermittler oder Puffer enthält.

Derartige Zusätze sind z.B. Tartrat- und Citratpuffer, Ethanol, Komplexbildner (wie Ethylendiamintetraessigsäure und deren nicht toxische Salze) und hochmolekulare Polymere (wie flüssiges Polyethylenoxid) zur Viskositätsregulierung. Feste Trägerstoffe sind z.B. Stärke, Lactose, Mannit, Methylcellulose, Talkum, hochdisperse Kieselsäuren, hochmolekulare Fettsäuren (wie Stearinsäure), Gelatine, Agar-Agar, Calciumphosphat, Magnesiumstearat, tierische und pflanzliche Fette und feste hochmolekulare Polymere (wie Polyethylenglykole). Für orale Applikation geeignete Zubereitungen können gewünschtenfalls Geschmacks- und Süßstoffe enthalten.

Die erfindungsgemäßen Verbindungen werden üblicherweise in Mengen von 10-2000 mg pro Tag bezogen auf 75 kg Körpergewicht appliziert. Bevorzugt ist es, 2-3 mal pro Tag 1-2 Tabletten mit einem Wirkstoffgehalt von 10-1500 mg zu verabreichen. Die Tabletten können auch retardiert sein, wodurch nur noch 1 mal pro Tag 1-2 Tabletten mit 1 -500 mg Wirkstoff gegeben werden muß. Der Wirkstoff kann auch durch Injektion 1-8 mal pro Tag bzw. durch Dauerinfusion gegeben werden, wobei Mengen von 10-1000 mg pro Tag normalerweise ausreichen.

Bevorzugt im Sinne der Erfindung sind außer den in den Beispielen genannten Verbindungen die folgenden:
2-(N-Oxy-3-pyridinyl)-7,7-dimethyl-5,7-dihydro-pyrrolo[3,2-f]benzoxazol-6-on
2-(N-Oxy-2-pyridinyl)-7,7-dimethyl-5,7-dihydro-pyrrolo[3,2-f]benzoxazol-6-on
2-(2-Chlor-4-pyridinyl)-7,7-dimethyl-5,7-dihydro-pyrrolo[3,2-f]benzoxazol-6-on
2-(2-Hydroxy-4-pyridinyl)-7,7-dimethyl-5,7-dihydro-pyrrolo[3,2-f]benzoxazol-6-on
2-(3-Hydroxy-4-pyridinyl)-7,7-dimethyl-5,7-dihydro-pyrrolo[3,2-f]benzoxazol-6-on
2-(2,6-Dihydroxy-4-pyridinyl)-7,7-dimethyl-5,7-dihydro-pyrrolo[3,2-f]benzoxazol-6-on
2-(6-Methyl-3-pyridinyl)-7,7-dimethyl-5,7-dihydro-pyrrolo[3,2-f]benzoxazol-6-on
2-(2-Methyl-5-pyrimidinyl)-7,7-dimethyl-5,7-dihydro-pyrrolo[3,2-f]benzoxazol-6-on
2-(5-Pyrimidinyl)-7,7-dimethyl-5,7-dihydro-pyrrolo[3,2-f]benzoxazol-6-on
2-(2-Hydroxy-5-pyrimidinyl)-7,7-dimethyl-5,7-dihydro-pyrrolo[3,2-f]benzoxazol-6-on
2-(3-Chinolinyl)-7,7-dimethyl-5,7-dihydro-pyrrolo[3,2-f]benzoxazol-6-on
2-(6-Hydroxy-3-pyridazinyl)-7,7-dimethyl-5,7-dihydro-pyrrolo[3,2-f]benzoxazol-6-on
2-(2-Indolyl)-7,7-dimethyl-5,7-dihydro-pyrrolo[3,2-f]benzoxazol-6-on
2-(3-Indolyl)-7,7-dimethyl-5,7-dihydro-pyrrolo[3,2-f]benzoxazol-6-on
2-(5-Methyl-3-pyrazolyl)-7,7-dimethyl-5,7-dihydro-pyrrolo[3,2-f]benzoxazol-6-on
2-(1,2,4-Triazol-3-yl)-7,7-dimethyl-5,7-dihydro-pyrrolo[3,2-f]benzoxazol-6-on
2-(1,2,3-Thiadiazol-5-yl)-7,7-dimethyl-5,7-dihydro-pyrrolo[3,2-f]benzoxazol-6-on
2-(2-Pyrrolyl)-7,7-dimethyl-5,7-dihydro-pyrrolo[3,2-f]benzoxazol-6-on
2-(4-Thiazolyl)-7,7-dimethyl-5,7-dihydro-pyrrolo[3,2-f]benzoxazol-6-on
2-(4-Imidazolyl)-7,7-dimethyl-5,7-dihydro-pyrrolo[3,2-f]benzoxazol-6-on
2-(1,2,5-Thiadiazol-3-yl)-7,7-dimethyl-5,7-dihydro-pyrrolo[3,2-f]benzoxazol-6-on
2-(5-Methylthio-1,3,4-oxadiazol-2-yl)-7,7-dimethyl-5,7-dihydro-pyrrolo[3,2-f]benzoxazol-6-on
2-(5-Methoxycarbonyl-1,2,3-triazol-4-yl)-7,7-dimethyl-5,7-dihydro-pyrrolo[3,2-f]benzoxazol-6-on
2-(5-Carboxy-1,2,3-triazol-4-yl)-7,7-dimethyl-5,7-dihydro-pyrrolo[3,2-f]benzoxazol-6-on
2-(2-Thenyl))-7,7-dimethyl-5,7-dihydro-pyrrolo[3,2-f]benzoxazol-6-on
2-(1-Methyl-1,2,3-triazol-4-yl)-7,7-dimethyl-5,7-dihydro-pyrrolo[3,2-f]benzoxazol-6-on
2-(4-Butyl-2-pyridinyl)-7,7-dimethyl-5,7-dihydro-pyrrolo[3,2-f]benzoxazol-6-on
2-(2-Benzofuranyl)-7,7-dimethyl-5,7-dihydro-pyrrolo[3,2-f]benzoxazol-6-on
2-(2-Indolizinyl)-7,7-dimethyl-5,7-dihydro-pyrrolo[3,2-f]benzoxazol-6-on
2-[2-(Benzimidazolyl)ethenyl]-7,7-dimethyl-5,7-dihydro-pyrrolo[3,2-f]benzoxazol-6-on
2-(5-Benzothiadiazolyl)-7,7-dimethyl-5,7-dihydro-pyrrolo[3,2-f]benzoxazol-6-on
2-(2-Thiopheno[2,3-f]pyrazinyl)-7,7-dimethyl-5,7-dihydro-pyrrolo[3,2-f]benzoxazol-6-on
2-(2-Methyl-5-thiazolyl)-7,7-dimethyl-5,7-dihydro-pyrrolo[3,2-f]benzoxazol-6-on
2-(3-Thienyl)-7,7-dimethyl-5,7-dihydro-pyrrolo[3,2-f]benzoxazol-6-on
2-(4-Pyridinyl)-7,7-diethyl-5,7-dihydro-pyrrolo[3,2-f]benzoxazol-6-on
2-(4-Pyridinyl)-7-methyl-5,7-dihydro-pyrrolo[3,2-f]benzoxazol-6-on
2-(4-Pyridinyl)-7-ethyl-5,7-dihydro-pyrrolo[3,2-f]benzoxazol-6-on
2-(4-Pyridinyl)-7-isopropyl-5,7-dihydro-pyrrolo[3,2-f]benzoxazol-6-on
2-(4-Pyridinyl)-7-(2-methylpropyl)-5,7-dihydro-pyrrolo[3,2-f]benzoxazol-6-on
2-(4-Pyridinyl)-7-cyclopentyl-5,7-dihydro-pyrrolo[3,2-f]benzoxazol-6-on
2-(4-Pyridinyl)-7-methyl-7-ethoxycarbonyl-5,7-dihydro-pyrrolo[3,2-f]benzoxazol-6-on
2-(4-Pyridinyl)-7-methyl-7-hydrazinocarbonyl-5,7-dihydro-pyrrolo[3,2-f]benzoxazol-6-on
2-(4-Pyridinyl)-5,7-dihydro-pyrrolo[3,2-f]benzoxazol-6-on
2′-(4-Pyridinyl)-5′,7′-dihydro-spiro[cyclopentan-1,7′-pyrrolo[3,2-f]benzoxazol]-6′-on
2-(2,3-Dimethyl-6-chinoxalinyl)-7,7-dimethyl-5,7-dihydro-pyrrolo[3,2-f]benzoxazol-6-on
2-(4-[1,8]-Naphthyridinyl)-7,7-dimethyl-5,7-dihydro-pyrrolo[3,2-f]benzoxazol-6-on
2-(4-Pyridinyl)-7,7-dipropyl-5,7-dihydro-pyrrolo[3,2-f]benzoxazol-6-on

### Beispiel 1

### 2-(4-Pyridinyl)-7,7-dimethyl-5,7-dihydro-pyrrolo[3,2-f]benzoxazol-6-on

4 g 1,3-Dihydro-3,3-dimethyl-6-hydroxy-5-nitro-2H-indol-2-on in 100 ml Eisessig in Gegenwart von 1 g 10 % Pd-C hydrierte man bei Raumtemperatur und Normaldruck, bis 1.3 l Wasserstoff aufgenommen waren. Man filtrierte, goß zum Filtrat 2.1 ml 4-Pyridincarbaldehyd, wobei sich die Lösung gelborgange färbte. Unter Rühren leitete man 3 d Luft durch die Lösung, engte i. Vak. zur Trockne ein, digerierte den Rückstand mit wässrigem Ammoniak und Wasser und reinigte säulenchromatographisch (800 ml Kieselgel, Laufmittel Dichlormethan: methanolischer Ammoniak = 20:1). Die entsprechenden Fraktionen wurden i. Vak. eingeengt, der kristalline Rückstand mit Ethanol digeriert und abgesaugt. Man erhielt 4.0 g beige Kristalle, die man aus Ethanol unter Bleicherdebehandlung umkristallisierte. Man erhielt 2.5 g der Titelverbindung als farblose Kristalle mit dem Fp. 336-338°C (49 % Ausbeute).

Die Vorstufe stellte man wie folgt her:

### 1,3-Dihydro-3,3-dimethyl-6-hydroxy-5-nitro-2H-indol-2-on

4.5 g 1,3-Dihydro-3,3-dimethyl-6-hydroxy-2H-indol-2-on wurde portionsweise in 35 ml 65 % HNO₃, die mit Eis/Kochsalzmischung gekühlt war, unter kräftigem Rühren eingetragen. Man rührte in der Kälte noch 10 min nach, verdünnte mit Eis/Wasser auf 150 ml, saugte den kristallinen Niederschlag ab und wusch mit Wasser nach. Man erhielt 4.1 g (73 %) der Titelverbindung als gelbe Kristalle mit dem Fp. 244-247°C.

### Beispiel 2

### 2-(4-Pyridinylmethyl)-7,7-dimethyl-5,7-dihydro-pyrrolo[3,2-f]benzoxazol-6-on

4.52 g 5-Amino-1,3-dihydro-3,3-dimethyl-6-hydroxy-2H-indol-2-on-Hydrochlorid und 3.65 g 4-Pyridylessigsäure erhitzte man in 20 g Polyphosphorsäure 30 min auf 170°C und goß anschließend auf Eis. Man gab konzentrierten wäßrigen Ammoniak bis pH = 10 zu, saugte den Niederschlag ab, wusch nacheinander mit Wasser, Methanol und Ether und trocknete bis zur Gewichtskonstanz. Man erhielt 3.20 g (55 %) der Titelverbindung mit dem Fp. 208-211°C.

Die Vorstufe stellte man wie folgt her:

### 5-Amino-1,3-dihydro-3,3-dimethyl-6-hydroxy-2H-indol-2-on-Hydrochlorid

22.2 g 1,3-Dihydro-3,3-dimethyl-6-hydroxy-5-nitro-2H-indol-2-on hydrierte man in 500 ml Methanol in Gegenwart von 5 g 10 % Pd auf Kohle. Nach 2.5 h waren 6.3 l H₂ aufgenommen, man filtrierte, dampfte das Filtrat zur Trockene ein, wusch den Rückstand mit Ether und trocknete bis zur Gewichtskonstanzs. man erhielt die Titelverbindung in 98 % Ausbeute mit dem FP. >250°C.

### Beispiel 3

### 2-(1,2,3-Thiadiazol-4-yl)-7,7-dimethyl-5,7-dihydro-pyrrolo[3,2-f]benzoxazol-6-on

1.5 g N-(1,3-Dihydro-3,3-dimethyl-6-hydroxy-2(2H)-oxo-indol-5-yl)-1,2,3-thiadiazol-4-carboxamid in 10 g Polyphosphorsäure erhitzte man 10 min auf 130°C, goß auf Eis, gab konzentrierte wäßrige Ammoniaklösung zu bis pH = 8-9, saugte den Niederschlag ab, wusch nacheinander mit Wasser, Methanol und Ether und trocknete bis zur Gewichtskonstanz. Man erhielt 1 g der Titelverbindung (71 %) mit dem Fp. >250°C.

Die Vorstufe stellte man wie folgt her:

### N-(1,3-Dihydro-3,3-dimethyl-6-hydroxy-2(2H)-oxo-indol-5-yl)-1,2,3-thiadiazol-4-carboxamid

Zu 1.4 g 5-Amino-1,3-dihydro-3,3-dimethyl-6-hydroxy-2H-indol-6-on-Hydrochlorid und 1,6 mL Triethylamin in 30 mL Dichlormethan tropfte man bei 0°C, 0.8 g 1,2,3-Thiadiazol-4-carbonsäure in 7 mL Dichlormethan. Nach 1 h Rühren bei Raumtemperatur saugte man den Niederschlag ab, wusch mit Wasser und trocknete bis zur Gewichtskonstanz. Man erhielt 1.5 g der Titelverbindung (92 % Ausbeute). Massenspektrum: m/e = 376 (M⁺ des TMS-Derivats).

### Beispiel 4

### 2-(2-Methoxy-6-methyl-3-pyridinyl)-7,7-dimethyl-5,7-dihydro-pyrrolo[3,2-f]benzoxazol-6-on

mit dem Fp. 236-238°C erhielt man analog dem Beispiel 1 in 54 % Ausbeute nach Chromatographie an Kieselgel (Essigester/Heptan = 1:1) durch Umsetzung mit 2-Methoxy-5-methyl-pyridinyl-3-aldehyd.

### Beispiel 5

### 2-(2-Thienyl)-7,7-dimethyl-5,7-dihydro-pyrrolo[3,2-f]benzoxazol-6-on

mit dem Fp. 276-280°C erhielt man analog dem Beispiel 1 in 35 % Ausbeute nach Umkristallisation aus Ethanol durch Umsetzung mit 2-Thiophenaldehyd.

### Beispiel 6

### 2-(2-Furyl)-7,7-dimethyl-5,7-dihydro-pyrrolo[3,2-f]benzoxazol-6-on

mit dem Fp. 240-245°C erhielt man analog dem Beispiel 1 in 25 % Ausbeute nach Umkristallisation aus Ethanol durch Umsetzung mit Furfural.

### Beispiel 7

### 2-(2-Imidazo[1,2-a]pyridinyl-methyl)-7,7-dimethyl-5,7-dihydro-pyrrolo[3,2-f]benzoxazol-6-on

mit dem Fp. 205-208°C erhielt man analog dem Beispiel 2 in 37 % Ausbeute durch Umsetzung mit 2-(2-Imidazo[1,2-a]pyridinyl-essigsäure.

### Beispiel 8

### 2-(2-(4-Pyridinyl)ethenyl)-7,7-dimethyl-5,7-dihydro-pyrrolo[3,2-f]benzoxazol-6-on

mit dem Fp. >250°C erhielt man analog dem Beispiel 2 in 60 % Ausbeute durch Umsetzung mit 3-(4-Pyridyl)acrylsäure.

### Beispiel 9

### 2-(2-(4-Pyridinyl)ethyl)-7,7-dimethyl-5,7-dihydro-pyrrolo[3,2-f]benzoxazol-6-on

mit dem Fp. 192-195°C erhielt man analog dem Beispiel 2 in 54 % Ausbeute durch Umsetzung mit 3-(4-Pyridinyl)propansäure.

### Beispiel 10

### 2-(2-Methyloxazol-4-yl)-7,7-dimethyl-5,7-dihydro-pyrrolo[3,2-f]benzoxazol-6-on

mit dem Fp. >250°C erhielt man analog dem Beispiel 2 in 3 % Ausbeute durch Umsetzung mit 2-Methyloxazol-4-carbonsäure.

### Beispiel 11

### 2-(4-Pyridazinyl)-7,7-dimethyl-5,7-dihydro-pyrrolo[3,2-f]benzoxazol-6-on

mit dem Fp. >250°C erhielt man analog dem Beispiel 2 in 39% Ausbeute durch Umsetzung mit 4-Pyridazincarbonsäure.

### Beispiel 12

### 2-(2-Pyrazinyl)-7,7-dimethyl-5,7-dihydro-pyrrolo[3,2-f]benzoxazol-6-on

mit dem Fp. >250°C erhielt man analog dem Beispiel 2 in 48 % Ausbeute durch Umsetzung mit 2-Pyrazincarbonsäure.

### Beispiel 13

### 2-(3-Pyridinyl)-7,7-dimethyl-5,7-dihydro-pyrrolo[3,2-f]benzoxazol-6-on

mit dem Fp. 285-287 °C erhielt man anlog dem Beispiel 1 in 10 % Ausbeute nach Umkristallisation aus Ethanol durch Umsetzung mit 3-Pyridinaldehyd.

### Beispiel 14

### 2-(2-Pyridinyl)-7,7-dimethyl-5,7-dihydro-pyrrolo[3,2-f]benzoxazol-6-on

mit dem Fp. 272-275 °C erhielt man analog dem Beispiel 1 in 10 % Ausbeute nach Umkristallisation aus Ethanol durch Umsetzung mit 2-Pyridinaldehyd.

### Beispiel 15

### 2-(4-Chinolinyl)-7,7-dimethyl-5,7-dihydro-pyrrolo[3,2-f]benzoxazol-6-on

mit dem Fp. >300 °C erhielt man analog dem Beispiel 1 in 13 % Ausbeute nach Umkristallisation aus Ethanol durch Umsetzung mit 4-Chinolinaldehyd.

### Beispiel 16

### 2-(N-Oxy-4-pyridinyl)-7,7-dimethyl-5,7-dihydro-pyrrolo[3,2-f]benzoxazol-6-on

Zu 4.0 g 2-(4-Pyridinyl)-7,7-dimethyl-5,7-dihydro-pyrrolo[3,2-f]benzoxazol-6-on in 80 ml Eisessig tropfte man 12 ml 30 % H₂O₂. Man rührte eine Woche bei Raumtemperatur, gab Wasser zu und stumpfte mit konz. Ammoniak bis pH = 6 ab. Man saugte das ausgefallene Produkt ab und reinigte säulenchromatographisch (Kieselgel 60, Dichlormethan/Methanol = 20:1). Die reinen Fraktionen wurden eingedampft und der Rückstand aus Ethanol/Wasser umkristallisiert. Man erhielt 1.4 g der Titelverbindung als blaßgelbe Kristalle mit dem Fp. 340-341°C.

## Patentansprüche

1. Verbindungen der Formel I 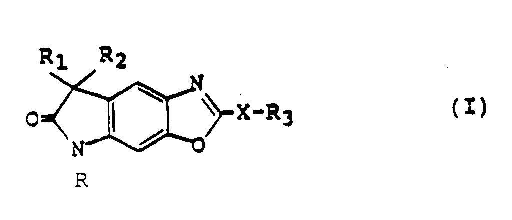 in der
R ein Wasserstoffatom oder eine C₁-C₆-Alkylgruppe darstellt,
R₁ ein Wasserstoffatom, eine C₁-C₆-Alkyl-, C₂-C₆-Alkenyl- oder eine C₃-C₈-Cycloalkylgruppe bedeutet,
R₂ ein Wasserstoffatom, eine C₁-C₆-Alkyl-, C₂-C₆-Alenyl, C₁-C₆-Alkylcarbonyl-, C₁-C₆-Alkoxycarbonyl-, Aminocarbonyl- oder Hydrazinocarbonylgruppe bedeutet, oder R₁ und R₂ zusammen mit dem C-Atom, an das sie gebunden sind, einen C₃-C₈-Spirocycloalkylring bilden,
X eine Bindung, eine C₁-C₆-Alkylen- oder die Vinylengruppe bedeutet,
R₃ einen aromatischen heterocyclischen Fünfring mit 1-4 Heteroatomen oder einen aromatischen heterocyclischen Sechsring mit 1-5 Heteroatomen darstellt, wobei die Heteroatome gleich oder verschieden sein können und Sauerstoff, Schwefel oder Stickstoff bedeuten und gewünschtenfalls an einem oder mehreren Stickstoffatomen ein Sauerstoffatom tragen können, und die Fünf- oder Sechringe gegebenenfalls durch eine oder mehrere C₁-C₆-Alkyl-, C₁-C₆-Alkoxy-, C₁-C₆-Alkoxycarbonyl-, Carboxyl-, C₁-C₆-Alkylmercapto-, Hydroxy-, Nitro-, Amino-, Halogen- oder Cyangruppen substituiert und gewünschtenfalls mit einem Phenylring oder einem aromatiscchen Fünf- oder Sechsring mit 1-4 Heteroatomen zu einem Bicyclus kondensiert sein können,
deren Tautomere, optisch aktive Formen und deren physiologisch verträgliche Salze anorganischer oder organischer Säuren.

2. Verbindungen der Formel I gemäß Anspruch 1,
in der
R ein Wasserstoffatom ist
R₁ ein Wasserstoffatom oder eine C₁-C₄-Alkylgruppe bedeutet,
R₂ ein Wasserstoffatom oder eine C₁-C₄-Alkylgruppe darstellt, oder R₁ und R₂ zusammen mit dem Kohlenstoffatom, an das sie gebunden sind, einen C₅-C₆-Spirocycloalkylring bilden,
X eine Bindung, eine C₁-C₄-Alkylen- oder Vinylengruppe darstellt, und
R₃ den Pyrrolyl-, Furanyl-, Thienyl-, Pyrazolyl-, Imidazolyl-, Isothiazolyl-, Thiazolyl-, Oxazolyl-, Triazolyl-, Tetrazolyl-, Thiadiazolyl-, Isoxazolyl-, Oxadiazolyl-, Pyridinyl-, N-Oxy-pyridinyl-, Pyrazinyl-, N,N′-Dioxypyrazinyl-, Pyrimidinyl-, N,N′-Dioxypyrimidinyl-, Pyridazinyl-, Oxazinyl-, Thiazinyl-, Triazinyl- oder Tetrazinylrest darstellt, sowie deren C₁-C₄-Alkyl-, C₁-C₄-Alkoxy-, C₁-C₄-Alkylmercapto- und Halogen- substituierten Derivate, oder R₃ den Indolyl-, Indazolyl-, Chinolinyl-, Isochinolinyl- oder Imidazo[1,2-a] pyridinylrest bedeutet.

3. Verbindungen der Formel I gemäß Anspruch 1 oder 2, in der R₁ und R₂ gleich sind und eine C₁-C₄-Alkylgruppe bedeuten.

4. Verbindungen der Formel I gemäß Anspruch 1 oder 2, in der R₃ einen Pyridinyl-, N-Oxy-pyridinyl-, Thiadiazolyl-, Thienyl-, Furyl-, Oxazolyl-, Pyridazinyl- oder Pyrazinylrest bedeutet, der durch C₁-C₄-Alkyl- oder C₁-C₄-Alkoxygruppen substituiert sein kann, oder R₃ einen Imidazopyridinyl- oder Chinolinylrest bedeutet.

5. Verbindungen der Formel I gemäß einem der Ansprüche 1 bis 4, in der R ein Wasserstoffatom ist.

6. Verbindungen der Formel I gemäß einem der Ansprüche 1-5, in der X eine Bindung, eine C₁-C₂-Alkylengruppe oder eine Vinylgruppe darstellt.

7. Verfahren zur Herstellung von Verbindung der Formel I gemäß den Ansprüchen 1 - 6,
dadurch gekennzeichnet, daß man in an sich bekannter Weise
a) eine Verbindung der allgemeinen Formel III 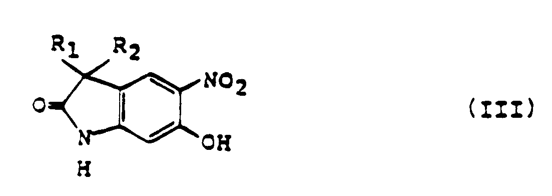 reduziert und mit einem Aldehyd der allgemeinen Formel IX
R₃ - X - CHO (IX)
oxidierend cyclisiert, oder mit einer Carbonsäure der allgemeinen Formel X
R₃ - X - Z (X)
reduzierend cyclisiert
b) eine Verbindung der allgemeinen Formel XVII 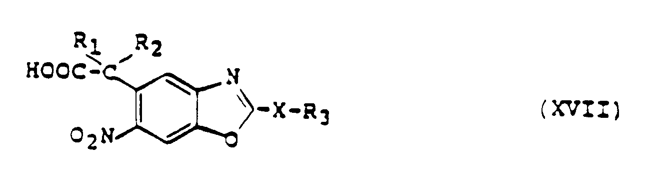 reduzierend cyclisiert,
c) eine Verbindung der allgemeinen Formel XXIII 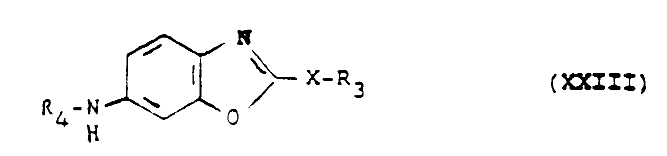 in der R₄ einen Rest der allgemeinen Formel XXIV 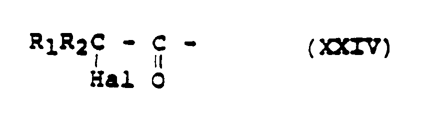 oder einen Rest der allgemeinen Formel XXV 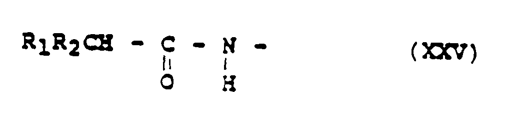 darstellt, cyclisiert,
und anschließend gewünschtenfalls die so erhaltenen Verbindungen der allgemeinen Formel I in andere Verbindungen der allgemeinen Formel I nachträglich umwandelt, sowie diese Verbindungen gegebenenfalls in ihre pharmakologisch verträglichen Salze überführt.

8. Arzneimittel, enthaltend mindestens eine Verbindung gemäß einem der Ansprüche 1 bis 6 sowie pharmazeutische Träger- und Hilfsstoffe.

9. Verwendung von Verbindungen gemäß einem der Ansprüche 1 bis 6 zur Herstellung von Arzneimittel mit immunomodulierenden Eigenschaften.

10. Verfahren zur Herstellung von Arzneimitteln enthaltend mindestens eine Verbindung der Formel I gemäß einem der Ansprüche 1 - 6, dadurch gekennzeichnet, daß man eine Verbindung der Formel I mit pharmazeutischen Träger- oder Hilfsstoffen vermischt und zu pharmazeutischen Darreichungsformen verarbeitet.

## Claims

1. Compounds of the formula I 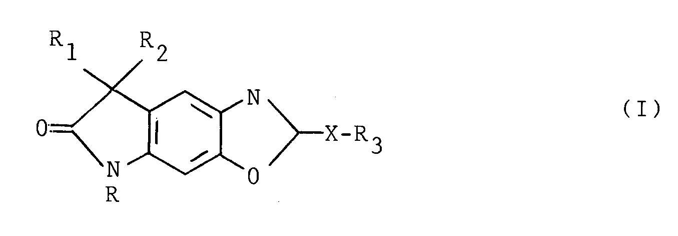 in which R represents a hydrogen atom or a C₁-C₆-alkyl group, R₁ signifies a hydrogen atom, a C₁-C₆-alkyl, C₂-C₆-alkenyl or a C₃-C₈-cycloalkenyl group, R₂ signifies a hydrogen atom, a C₁-C₆-alkyl, C₂-C₆-alkenyl, C₁-C₆-alkylcarbonyl, C₁-C₆-alkoxycarbonyl, aminocarbonyl or hydrazinocarbonyl group or R₁ and R₂, together with the C-atom to which they are attached, form a C₃-C₈-spirocycloalkyl ring, X signifies a bond, a C₁-C₆-alkylene or the vinylene group, R₃ represents an aromatic heterocyclic five-membered ring with 1 - 4 heteroatoms or an aromatic heterocyclic six-membered ring with 1 - 5 heteroatoms, whereby the heteroatoms can be the same or different and signify oxygen, sulphur or nitrogen and, if desired, can carry an oxygen atom on one or more nitrogen atoms and the five- or six-membered rings can be optionally substituted by one or more C₁-C₆-alkyl, C₁-C₆-alkoxy, C₁-C₆-alkoxycarbonyl, carboxyl, C₁-C₆-alkylmercapto, hydroxyl, nitro, amino, halogen or cyano groups and, if desired, can be condensed with a phenyl ring or an aromatic five- or six-membered ring with 1 - 4 heteroatoms to form a bicyclic radical; their tautomers, optically-active forms and their physiologically compatible salts of inorganic or organic acids.

2. Compounds of the formula I according to claim 1, in which R is a hydrogen atom, R₁ signifies a hydrogen atom or a C₁-C₄-alkyl group, R₂ represents a hydrogen atom or a C₁-C₄-alkyl group or R₁ and R₂, together with the carbon atom to which they are attached, form a C₅-C₆-spirocycloalkyl ring, X represents a bond, a C₁-C₄-alkylene or vinylene group and R₃ represents the pyrrolyl, furanyl, thienyl, pyrazolyl, imidazolyl, isothiazolyl, thiazolyl, oxazolyl, triazolyl, tetrazolyl, thiadiazolyl, isoxazolyl, oxadiazolyl, pyridinyl, N-oxypyridinyl, pyrazinyl, N,N'-dioxypyrazinyl, pyrimidinyl, N,N'-dioxypyrimidinyl, pyridazinyl, oxazinyl, thiazinyl, triazinyl or tetrazinyl radical, as well as their C₁-C₄-alkyl-, C₁-C₄-alkoxy-, C₁-C₄-alkylmercapto- or halogen-substituted derivatives or R₃ signifies the indolyl, indazolyl, quinolinyl, isoquinolinyl or imidazo[1,2-a]pyridinyl radical.

3. Compounds of the formula I according to claim 1 or 2 in which R₁ and R₂ are the same and signify a C₁-C₄-alkyl group.

4. Compounds of the formula I according to claim 1 or 2, in which R₃ signifies a pyridinyl, N-oxypyridinyl, thiadiazolyl, thienyl, furyl, oxazolyl, pyridazinyl or pyrazinyl radical which can be substituted by C₁-C₄-alkyl or C₁-C₄-alkoxy groups or R₃ signifies an imidazopyridinyl or quinolinyl radical.

5. Compounds of the formula I according to one of claims 1 to 4 in which R is a hydrogen atom.

6. Compounds of the formula I according to one of claims 1 - 5 in which X is a bond, a C₁-C₂-alkylene group or a vinyl group.

7. Process for the preparation of compounds of the formula I according to claims 1 - 6, characterised in that, in per se known manner, one
a) reduces a compound of the general formula III 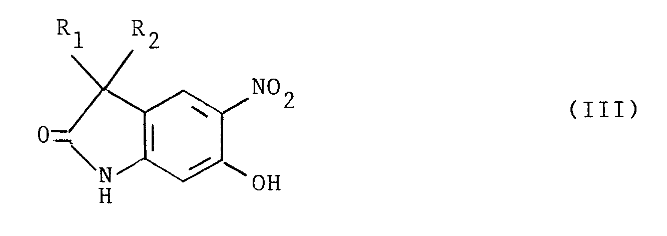 and oxidisingly cyclises with an aldehyde of the general formula IX
R₃ - X - CHO (IX)
or reducingly cyclises with a carboxylic acid of the general formula X
R₃ - X - Z (X)
b) reducingly cyclises a compound of the general formula XVII 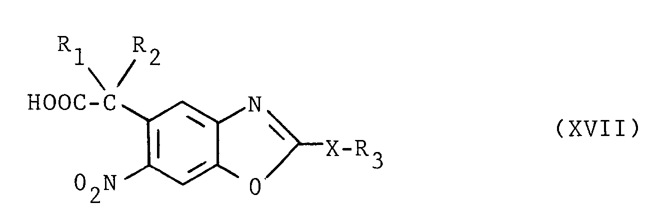
c) cyclises a compound of the general formula XXIII 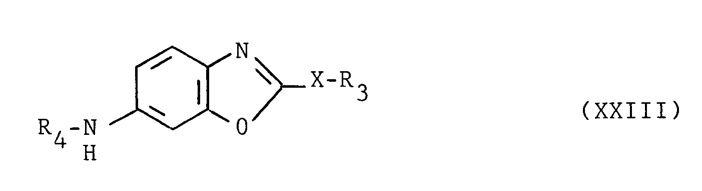 in which R₄ represents a radical of the general formula XXIV 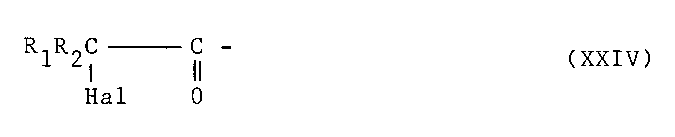 or a radical of the general formula XXV 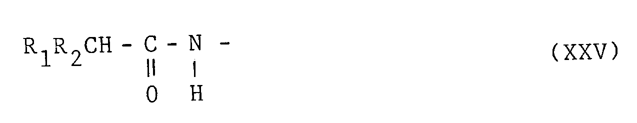 and subsequently, if desired, converts the so-obtained compounds of the general formula I into other compounds of the general formula I, as well as, if desired, converts these compounds into their pharmacologically compatible salts.

8. Medicaments containing at least one compound according to one of claims 1 to 6, as well as pharmaceutical carrier and adjuvant materials.

9. Use of compounds according to one of claims 1 to 6 for the preparation of medicaments with immune-modulating properties.

10. Process for the preparation of medicaments containing at least one compound of the formula I according to one of claims 1 - 6, characterised in that one mixes a compound of the formula I with pharmaceutical carrier or adjuvant materials and works up to pharmaceutical forms of administration.

## Revendications

1. Composés de formule I 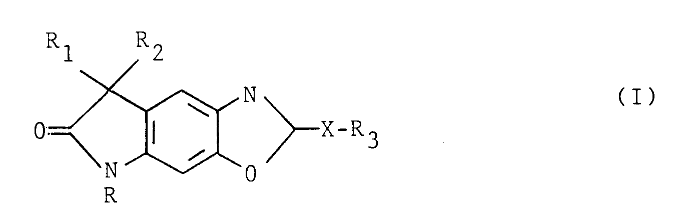 dans laquelle
R représente un atome d'hydrogène ou un groupe alkyle en C₁-C₆,
R₁ représente un atome d'hydrogène, un groupe alkyle en C₁-C₆, alcényle en C₂-C₆ ou cycloalkyle en C₃-C₈,
R₂ représente un atome d'hydrogène, un groupe alkyle en C₁-C₆, alcényle en C₂-C₆, alkyl(C₁-C₆)carbonyle, alcoxy(C₁-C₆)carbonyle, aminocarbonyle ou hydrazinocarbonyle, ou R₁ et R₂ forment, conjointement avec l'atome de C auquel ils sont liés, un noyau spirocycloalkyle en C₃-C₈,
X représente une valence libre, un groupe alkylène en C₁-C₆ ou un groupe vinylène,
R₃ représente un noyau hétérocyclique aromatique à cinq chaînons, comportant 1-4 hétéroatomes, ou un noyau hétérocyclique aromatique à six chaînons, comportant 1-5 hétéroatomes, les hétéroatomes pouvant être identiques ou différents et signifier un atome d'oxygène, de soufre ou d'azote, et éventuellement, un ou plusieurs atomes d'azote pouvant porter un atome d'oxygène, et le noyau à cinq ou six chaînons peut être éventuellement substitué par un ou plusieurs groupes alkyle en C₁-C₆, alcoxy en C₁-C₆, alcoxy(C₁-C₆)carbonyle, carboxyle, alkyl(C₁-C₆)mercapto, hydroxy, nitro, amino, halogéno ou cyano, et éventuellement, ils peuvent être condensés avec un noyau phényle ou un noyau aromatique à cinq ou six chaînons comportant 1-4 hétéroatomes, pour donner un système bicyclique,
leurs tautomères, leurs formes optiquement actives et leurs sels physiologiquement acceptables, formés avec des acides minéraux ou organiques.

2. Composés de formule I selon la revendication 1,
dans laquelle
R représente un atome d'hydrogène,
R₁ représente un atome d'hydrogène ou un groupe alkyle en C₁-C₄,
R₂ représente un atome d'hydrogène ou un groupe alkyle en C₁-C₄, ou R₁ et R₂ forment, conjointement avec l'atome de carbone auquel ils sont liés, un noyau spirocycloalkyle en C₅-C₆,
X représente une valence libre, un groupe alkylène en C₁-C₄ ou un groupe vinylène, et
R₃ représente un groupement pyrrolyle, furanyle, thiényle, pyrazolyle, imidazolyle, isothiazolyle, thiazolyle, oxazolyle, triazolyle, tétrazolyle, thiadiazolyle, isoxazolyle, oxadiazolyle, pyridinyle, N-oxypyridinyle, pyrazinyle, N,N'-dioxypyrazinyle, pyrimidinyle, N,N'-dioxypyrimidinyle, pyridazinyle, oxazinyle, thiazinyle, triazinyle ou tétrazinyle, ainsi que leurs dérivés substitués par un groupe alkyle en C₁-C₄, alcoxy en C₁-C₄, alkyl(C₁-C₄)mercapto et halogéno, ou R₃ représente le groupe indolyle, indazolyle, quinolinyle, isoquinolinyle ou imidazo[1,2-a]pyridinyle.

3. Composés de formule I selon la revendication 1 ou 2, dans laquelle R₁ et R₂ sont identiques et représentent un groupe alkyle en C₁-C₄.

4. Composés de formule I selon la revendication 1 ou 2, dans laquelle R₃ représente un groupe pyridinyle, N-oxypyridinyle, thiadiazolyle, thiényle, furyle, oxazolyle, pyridazinyle ou pyrazinyle, qui peut être substitué par un groupe alkyle en C₁-C₄ ou alcoxy en C₁-C₄, ou R₃ représente un groupe imidazopyridinyle ou quinolinyle.

5. Composés de formule I selon l'une quelconque des revendications 1 à 4, dans laquelle R représente un atome d'hydrogène.

6. Composés de formule I selon l'une quelconque des revendications 1 à 5, dans laquelle X représente une valence libre, un groupe alkylène en C₁-C₂ ou un groupe vinyle.

7. Procédé de préparation des composés de formule I selon les revendications 1 à 6,
caractérisé en ce que, d'une manière connue en soi,
a) on réduit un composé de formule générale III 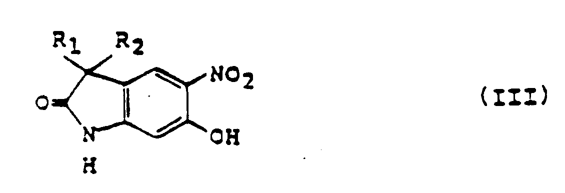 et on le soumet à une cyclisation oxydante avec un aldéhyde de formule générale IX
R₃ - X - CHO (IX)
ou on le soumet à une cyclisation réductrice avec un acide carboxylique de formule générale X
R₃ - X - Z (X)
b) on soumet un composé de formule générale XVII 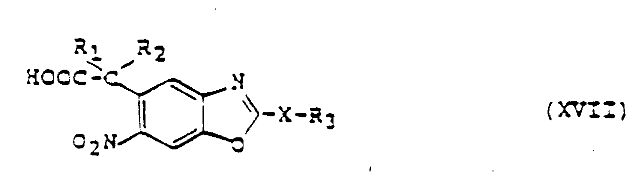 à une cyclisation réductrice,
c) on cyclise un composé de formule générale XXIII 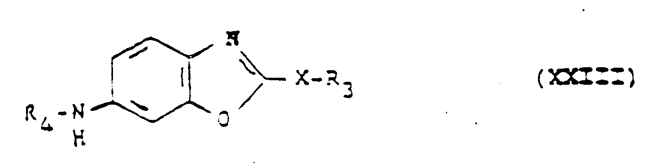 dans laquelle R₄ représente un groupe de formule générale XXIV 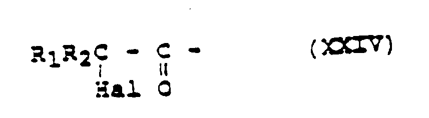 ou un groupe de formule générale XXV 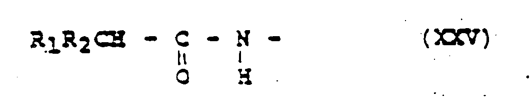 et ensuite, on transforme éventuellement les composés de formule générale I ainsi obtenus en d'autres composés de formule générale I, et l'on transforme éventuellement ces composés en leurs sels pharmacologiquement acceptables.

8. Médicaments contenant au moins un composé selon l'une quelconque des revendications 1 à 6, ainsi que des véhicules et adjuvants pharmaceutiques.

9. Utilisation de composés selon l'une quelconque des revendications 1 à 6 pour la préparation de médicaments ayant des propriétés immunomodulatrices.

10. Procédé de préparation de médicaments contenant au moins un composé de formule I selon l'une quelconque des revendications 1 à 6, caractérisé en ce que l'on mélange un composé de formule I avec des véhicules ou adjuvants pharmaceutiques et transforme ce mélange en une forme galénique.
